(19) **Europäisches Patentamt**
**European Patent Office**
**Office européen des brevets**

(11) **EP 2 298 176 A1**

(12) **EUROPEAN PATENT APPLICATION**
published in accordance with Art. 153(4) EPC

(43) Date of publication:
**23.03.2011 Bulletin 2011/12**

(21) Application number: **09758308.2**

(22) Date of filing: **02.06.2009**

(51) Int Cl.:
**A61B 8/08** (2006.01)  **A61B 6/00** (2006.01)

(86) International application number:
**PCT/JP2009/060043**

(87) International publication number:
**WO 2009/148041 (10.12.2009 Gazette 2009/50)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **03.06.2008 JP 2008145456**

(71) Applicant: **Hitachi Medical Corporation Chiyoda-ku Tokyo 101-0021 (JP)**

(72) Inventor: **CHONO,Tomoaki Tokyo 101-0021 (JP)**

(74) Representative: **Strehl Schübel-Hopf & Partner Maximilianstrasse 54 80538 München (DE)**

(54) **MEDICAL IMAGE PROCESSING DEVICE AND METHOD FOR PROCESSING MEDICAL IMAGE**

(57) An ultrasonic diagnostic apparatus in accordance with the invention includes: an image information obtaining section configured to obtain multiple medical images in which a living tissue of a subject to be tested is imaged; and an image recognition calculation section configured to obtain motion information of the living tissue from pixel values of medical images at different time phases of the obtained multiple medical images and classify the medical images into predetermined types based on the motion information.

FIG. 2

(a)  (b)  (c)  (d)  (e)

**Description**

**Technical Field**

The present invention relates to a medical image processing device and a medical image processing method for classifying a medical image according to the type of cross-section information and tissue motion of the medical image using image recognition.

**Background Art**

In image diagnosis using a medical image diagnostic apparatus, in order for a tester (operator) to efficiently detect a disease or perform other diagnoses, a medical image is classified according to the type of cross-section information and tissue motion of the medical image.

This classification method classifies a medical image using an image recognition technique based on the fact that a feature of cross-section information and tissue motion can be found from the shape of the image. It is more efficient for the tester to read previously classified images than classifying and reading medical images one by one.

For example, in measuring the heart, an ultrasonic diagnostic apparatus performs classification by obtaining the type of a cross section and the location information of a tissue of the heart using an image recognition technique utilizing a feature quantity of the luminance value of a still image. Further, in measuring the heart, the ultrasonic diagnostic apparatus performs motion analysis on a living tissue and displays the analysis result so that the tester can correct a misclassified medical image (for example, see Patent Document 1).

**Prior Art Document**

Patent Document

Patent Document 1: JP-A-2002-140689

**Summary of the Invention**

Problems to be Solved by the Invention

However, Patent Document 1 only discloses that motion analysis is performed on the living tissue, then the analysis result is displayed, and then the tester corrects a misclassified medical image.

So, Patent Document 1 does not consider improving the classification accuracy of medical images including those of moving living tissues.

It is an object of the present invention to provide a medical image processing device and a medical image processing method that can improve the classification accuracy of medical images including those of moving living tissues.

**Means for Solving the Problems**

In order to achieve the above object, an medical image processing device in accordance with the invention is characterized by including: an image information obtaining section configured to obtain multiple medical images in which a living tissue of a subject to be tested is imaged; and an image recognition calculation section configured to obtain motion information of the living tissue from pixel values of medical images at different time phases of the obtained multiple medical images and classifying the medical images into predetermined types based on the motion information.

The image information obtaining section obtains multiple medical images in which a living tissue of the subject to be tested is imaged, then the image recognition calculation section obtains motion information of the living tissue from pixel values of medical images at different time phases of the obtained multiple medical images and classifies the medical images into predetermined types based on the motion information, enabling improving the accuracy of image recognition of the medical images.

Furthermore, a medical image processing method in accordance with the invention is characterized by including: a step in which an image information obtaining section obtains multiple medical images in which a living tissue of a subject to be tested is imaged; and a step in which an image recognition calculation section obtains motion information of the living tissue from pixel values of medical images at different time phases of the obtained multiple medical images and classifies the medical images into predetermined types based on the motion information.

In the medical image obtaining step, the image information obtaining section obtains multiple medical images in which a living tissue of the subject to be tested is imaged, then in the medical image classifying step, the image recognition calculation section obtains motion information of the living tissue from pixel values of medical images at different time phases of the obtained multiple medical images and classifies the medical images into predetermined types based on the motion information, enabling improving the accuracy of image recognition of the medical images.

### Advantage of the Invention

According to the invention, a medical image processing device and a medical image processing method that can improve the classification accuracy of medical images including those of moving living tissues can be provided.

### Brief Description of the Drawings

[Fig. 1] A schematic block diagram of a medical image processing device in accordance with a first embodiment of the invention.
[Fig. 2] An illustration showing basic cross sections obtained by echocardiography and an example of extracting luminance value of an image and motion vector of a tissue.
[Fig. 3] A flowchart schematically showing an operation of a medical image processing device in accordance with a second embodiment of the invention.
[Fig. 4] A diagram showing the principle of the segmentation of the area of heart muscle.
[Fig. 5] A flowchart schematically showing an operation of a medical image processing device in accordance with a third embodiment of the invention.
[Fig. 6] A schematic block diagram of a medical image processing device in accordance with a fourth embodiment of the invention.
[Fig. 7] A schematic block diagram of a medical image processing device in accordance with a fifth embodiment of the invention.
[Fig. 8] A schematic block diagram of a medical image processing device in accordance with a fifth embodiment of the invention.
[Fig. 9] A flowchart schematically showing an operation of the medical image processing device in accordance with the fifth embodiment of the invention.
[Fig. 10] An illustration showing a display example of a stress echo test for explaining a sixth embodiment of the invention.
[Fig. 11] A flowchart schematically showing an operation of image search of a medical image processing device in accordance with a seventh embodiment of the invention.
[Fig. 12] An illustration showing a display example in the case that an image most similar to an image from the medical image generator is displayed.
[Fig. 13] An illustration showing a display example in the case that two or more images are similar to an image from the medical image generator.
[Fig. 14] An illustration showing an example of vector calculation method different from that of Figs. 2 and 5.

### Mode for Carrying Out the Invention

Embodiments of the invention are described in detail below with reference to the drawings.
In the description, an ultrasonic image obtained from an ultrasonic diagnostic apparatus is taken as an example of a medical image. Also, an image of the heart is taken as an example of an image of a living tissue in motion.
In an example to be described below, the ultrasonic diagnostic apparatus variably sets a time period for extracting the motion of the heart, and classifies ultrasonic images into predetermined types during the variably set time period for extracting the motion of the heart.

### First Embodiment

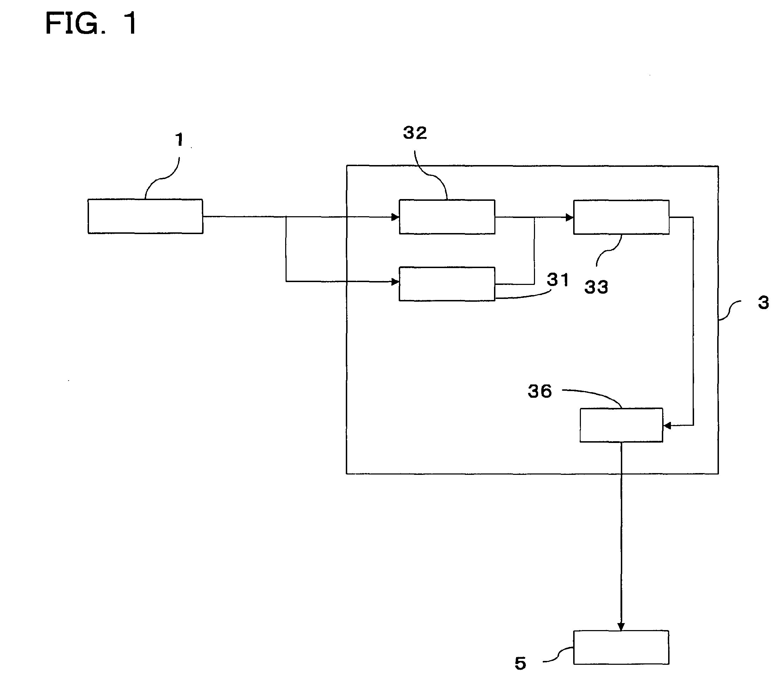

Fig. 1 is a schematic block diagram of a medical image processing device in accordance with a first embodiment of the invention.
As shown in Fig. 1, the medical image processing device includes: an ultrasonic image generator 1; an image recognition calculation section 3 communicatably connected to the ultrasonic image generator 1; and an image display 5 communicatably connected to the image recognition calculation section 3. As used herein, "communicatably connected" means that a connecting body and a connected body are connected by any means such as electromagnetic wave or light so that signals can be communicated therebetween.

The ultrasonic image generator 1 generates ultrasonic images from the ultrasonic diagnostic apparatus. The ultrasonic images include a B-mode image (tomography image), M-mode image and 3D-mode image.

The image recognition calculation section 3 identifies a type of ultrasonic image. Specifically, the image recognition calculation section 3 receives an input image from the ultrasonic image generator 1, extracts a feature quantity of the motion of the entire input image, and classifies the type of the cross-section. The image recognition calculation section 3 further includes: a motion extraction calculation section 31, communicatably connected to the ultrasonic image generator 1, for extracting the motion of a living tissue; an luminance extraction calculation section 32, communicatably connected to the ultrasonic image generator 1, for extracting the luminance of the living tissue; a feature extraction calculation section 33, communicatably connected to both of the motion extraction calculation section 31 and the luminance extraction calculation section 32, for calculating the extracted quantities of the extracted motion and storing them in a memory (not shown) thereof; and an identification calculation section 36, communicatably connected to the feature extraction calculation section 33, for identifying the type of the input ultrasonic image based on the feature quantity.

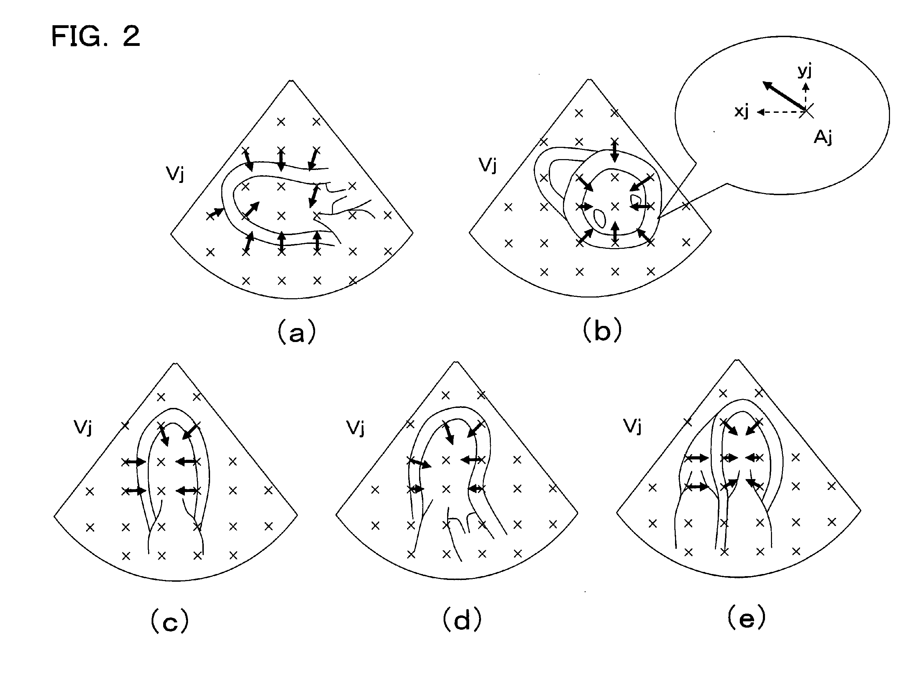

There are five basic types of cross sections obtained by echocardiography. Fig. 2 shows the basic cross sections obtained by echocardiography. As used herein, the basic cross sections include (a) parasternal long-axis view, (b) parasternal short-axis view, (c) apical two chamber view, (d) apical long axis view, and (e) apical four chamber view, as shown in Fig. 2.

Classification into the above types of images automatically performed using an image recognition technique rather than manually performed by a tester would be useful in reducing the amount of operation of the tester when diagnosing in image measurement.

For the motion of the heart, the motion extraction calculation section 31 performs motion extraction calculation on an extraction area to calculate the motion as a group of motion vectors of individual points, for example, by averaging. For example, a motion calculation method using block-matching method or gradient method is used.

In motion detection using block-matching method, first, in one of two consecutive frames of interest, a small area including the image of an object for motion detection is stored as a block. Next, in the other frame, image similarity calculation is performed on an area having the same size as the above block. This image similarity calculation is performed on all the areas of the frame. Then, the area with the most similarity in the frame would be an area to which the image of the object for motion detection has been moved. So, the moving distance and moving direction are calculated from the coordinates of the area with the most similarity and the coordinates of the previously stored block, and the process ends. Block-matching method determines the similarity to the target block for all the areas of the frame, which requires enormous amount of calculation but provides accurate motion detection.

On the other hand, gradient method searches for a corresponding point utilizing binding of luminance gradient of individual pixels in a space-time. This method is based on the assumption that the gray pattern of the image remains unchanged while in motion, and performs analysis based on an equation relating the spatial gradient and the temporal gradient of the gray pattern in the image. Gradient method requires less amount of calculation and less processing time, but tends to be noisy when determining the flow of an object that moves dynamically.

Since an individual difference in heart rate exists among subjects to be tested, the time period over which the motion of the heart is calculated should be freely settable by a tester. For example, for this calculation time period, a time period during which the amount of motion is largest, such as from end-diastole to end-systole, may be extracted. If a next image data exists, the luminance value and motion are extracted similarly.

Furthermore, Fig. 2 shows an example of a motion vector of the entire image from end-diastole to end-systole (a first motion vector). Here, motion vector shows the amount and direction of movement of the luminance value $A_j$ between different time phases. Motion vector can be expressed by horizontal movement component $x_j$ (in X-direction of the figure) and vertical movement component $y_j$ (in Y-direction of the figure).

A feature quantity of luminance value and a feature quantity of motion in each cross section can be obtained by calculating this motion vector.

Here, a vector $A_j$ represents a vector in the j-th image, and $V_j$ represents the type of the cross section. The motion vector $A_j$ is decomposed in X- and Y-directions of the figure into $x_j$ and $y_j$, respectively. In polar coordinate system rather than Cartesian coordinate system, the motion vector $A_j$ may be decomposed into radius $r_j$ and angle $\theta_j$. Thus, information $l_j$ obtained from an image j can be expressed as vector by Eq. 1.

$$l_j = (x_j | y_j) \qquad (\text{Eq. 1})$$

When used in conjunction with the luminance, the information $l_j$ can be expressed by Eq. 2 using the output of the luminance extraction calculation section 32. For example, the luminance extraction calculation section 32 outputs the amplitude of the ultrasonic signal as morphological information of the living tissue.

$$Ij = (Aj|xj|yj) \qquad (Eq.\ 2)$$

The combined use of motion and luminance may improve the accuracy of identifying the basic cross sections in echocardiography.

The feature extraction calculation section 33 extracts from the information lj of the entire image the feature quantity of each cross section V to be depicted as an image. For example, the feature extraction calculation section 33 extracts the feature quantity of each cross section Vj to be depicted as an image by applying a method using calculation of basic statistics, such as average or variance, or principal component analysis or independent component analysis of the motion vector Aj in each pixel of the entire ultrasonic image. The target of principal component analysis or the like is each pixel xj, yj of the entire ultrasonic image when the motion vector Aj is expressed by x and y coordinates or each pixel rj, θj of the entire ultrasonic image when the motion vector Aj is expressed by polar coordinates.

The identification calculation section 36 reads the feature quantity of the each cross section V from the memory and identifies the type of the input ultrasonic image from the read feature quantity. Specifically, in the example shown in Fig. 2(c), the identification calculation section 36 identifies the type of the ultrasonic image as apical two chamber view from the feature quantity.

The image display 5 displays by type the ultrasonic images with their types identified. Specifically, the example shown in Fig. 2(C), the image display 5 displays the apical two chamber view with the type identified.

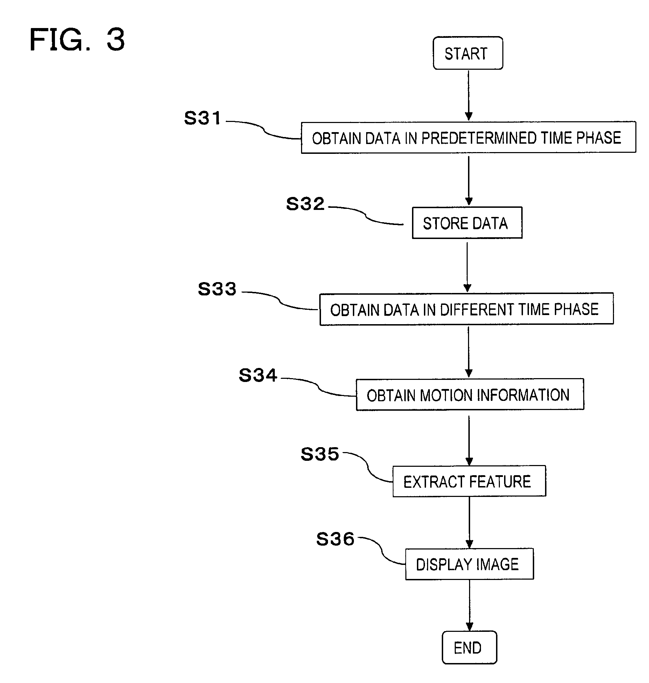

Fig. 3 is a flowchart schematically showing an operation of the medical image processing device in accordance with the first embodiment of the invention.

The medical image generator 1 obtains one frame of ultrasonic image in a predetermined time phase (step S31).

The image recognition calculation section 3 stores the ultrasonic image in a storage section (not shown) thereof (step S32).

The medical image generator 1 obtains one frame of ultrasonic image in a different time phase from the predetermined time phase (e.g., a time phase right after the predetermined time phase) (step S33).

The image recognition calculation section 3 uses the above-described method to obtain a motion vector from the ultrasonic image in the different time phase and the stored ultrasonic image. The motion extraction calculation section 31 of the image recognition calculation section 3 analyzes the directional components of the obtained motion vector (step S34).

The feature extraction calculation section 33 of the image recognition calculation section 3 extracts the feature quantity of each cross section V from the information lj by applying, for example, a method using calculation of basic statistics, such as average or variance, or principal component analysis or independent component analysis. The identification calculation section 36 identifies the basic cross sections in echocardiography from the feature quantity and generates a display format associating the basic cross-section information with the ultrasonic image (step S35).

The image display 5 displays the basic cross-section information and the ultrasonic image side by side according to the display format. Note that this display step is not necessary when the ultrasonic image is classified and stored in the storage section without being displayed (step S36).

This embodiment has the effect of improving the classification accuracy of medical images including those of moving living tissues. Furthermore, this embodiment has the specific effect of enabling real-time classification of the ultrasonic image in the different time phase due to the minimum configuration and the shortest path for processing the ultrasonic image.

## Second Embodiment

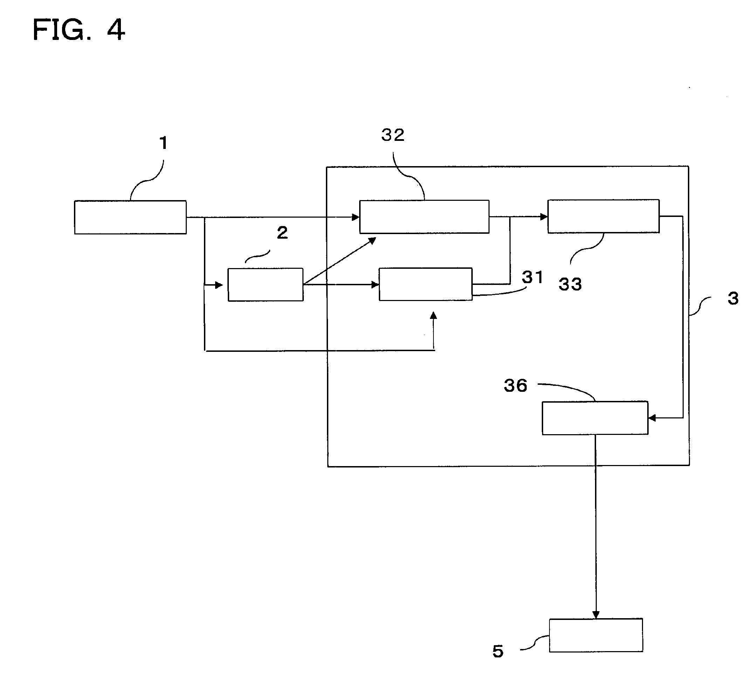

Fig. 4 is a schematic block diagram of a medical image processing device in accordance with a second embodiment of the invention.

In addition to the configuration shown in Fig. 1, the medical image processing device further includes a motion information obtaining section 2 communicatably connected to the ultrasonic image generator 1, as shown in Fig. 4.

The motion information obtaining section 2 divides the area of heart muscle of the heart on the ultrasonic image through program execution by a computer. The computer includes: a storage section (not shown) for storing data such as ultrasonic image from the ultrasonic image generator 1; and a CPU (not shown) for executing the computer program to process data and outputting the processed data to the image recognition calculation section 3.

The image recognition calculation section 3 receives local images segmented into fractions output from the motion information obtaining section 2 and outputs classified information.

At this point, the tester can set (select) either the entire image or a local area of the image. As a specific example, the tester selects the entire image when entirely diagnosing the motion of all organs and selects a local area of the image when diagnosing partial calcification or ischemic information of the heart muscle.

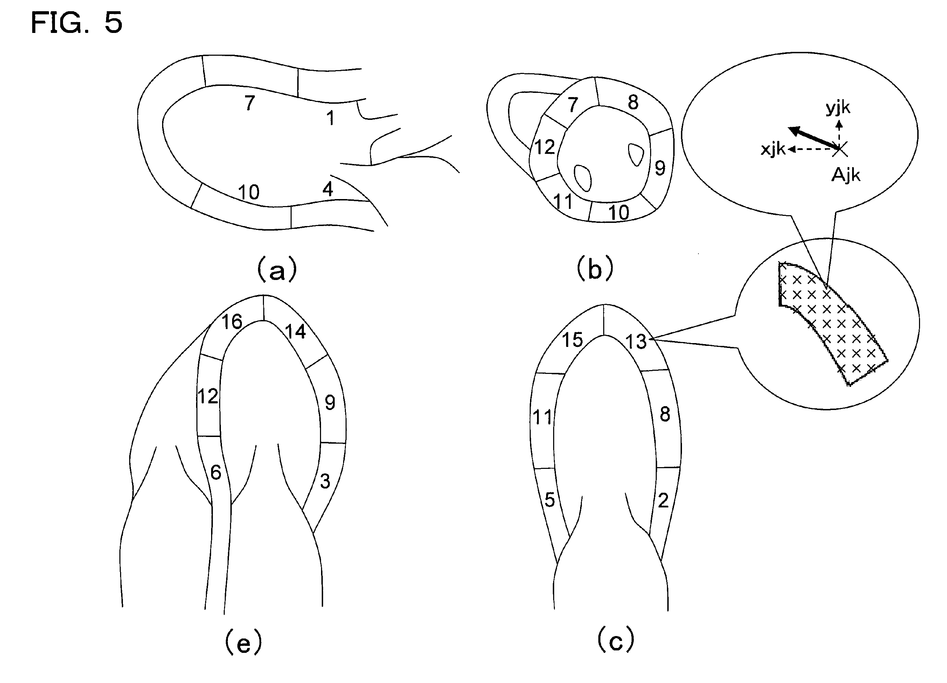

Fig. 5 shows the principle of the segmentation of the area of heart muscle. The segmentation of the area of heart muscle refers to segmenting the heart muscle into 16 or 17 fractions according to the recommendation of ASE (American Society of Echocardiography), as shown in Fig. 5. This segmentation is performed manually by the tester or by image recognition processing using the shape of the image as a feature quantity.

Here, a motion vector of a local area of the image (a second motion vector) is shown as an example. This motion vector shows the amount and direction of movement of the luminance value Aj between different time phases. This motion vector can be expressed by horizontal movement component xj (in X-direction of the figure) and vertical movement component yj (in Y-direction of the figure), as with the first motion vector. The difference from the first motion vector is that the first motion vector is motion analysis information from the entire image, whereas the second motion vector is motion analysis information only from the 13-th fractional image (local image) of (c) apical two chamber view.

Here, Ajk represents a vector in which luminance values are arranged in any order of extraction locations in the k-th fraction of the j-th image.

It can be considered that the motion vector of the entire image from end-diastole to end-systole of the heart differs between the cross sections. Here, Aj represents a vector in which luminance values are arranged in any order of extraction locations in the j-th pixel of the image. Also, Vj represents the type of the cross section. The motion vector is decomposed into orthogonal components xj, yj. In polar coordinate system, the motion vector may be decomposed into radius rj and angle θj. Thus, information lj obtained from an image j can be expressed as vector by Eq. 2 mentioned above.

When only luminance is to be used,

$$Ij = (Aj) \qquad (Eq. \ 3)$$

may be used. When only motion is to be used, Eq. 1 mentioned above may be used.

The motion vector is decomposed into orthogonal components xjk, yjk. In polar coordinate system, the motion vector may be decomposed into radius rjk and angle θjk. Thus, information ljk obtained from a fraction k of an image j can be expressed as vector by Eq. 4.

$$Ijk = (Ajk|xjk|yjk) \qquad (Eq. \ 4)$$

When only luminance is to be used, Eq. 5 may be used. When only motion is to be used, Eq. 6 may be used.

$$Ijk = (Ajk) \qquad (Eq. \ 5)$$

$$Ijk = (xjk|yjk) \qquad (Eq. \ 6)$$

It may be said that the first motion vector is a macroscopic motion analysis of an entire sector image. The accuracy of macroscopic classification may need further improvement. Thus, the second motion vector may be combined with and complement the first motion vector. The second motion vector is a microscopic motion analysis of segmented images of the sector image.

This embodiment is the same as the first embodiment except that the following program is executed in steps S32 and S34.

The motion information obtaining section 2 stores the ultrasonic image in a storage section thereof (step S32).

The motion information obtaining section 2 uses the above-described method to obtain a motion vector from the ultrasonic image in the different time phase and the stored ultrasonic image. The motion extraction calculation section 31 of the image recognition calculation section 3 analyzes the directional components of the obtained motion vector (step S34).

This embodiment has the effect of improving the classification accuracy of medical images including those of moving living tissues. Furthermore, this embodiment has the specific effect of enabling analysis of an abnormality in motion of a local area of an organ using the motion information obtaining section 2.

**Third Embodiment**

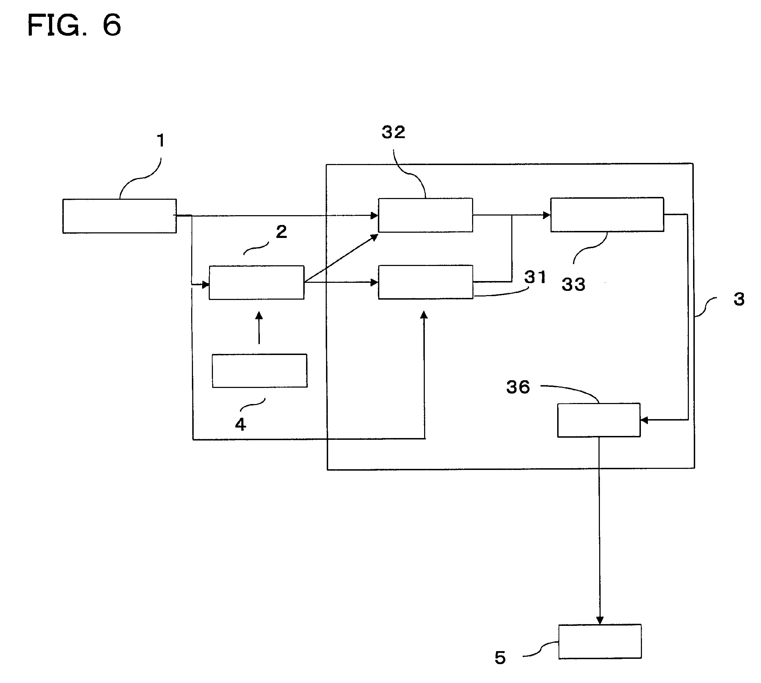

In addition to the configuration of the second embodiment, the medical image processing device further includes an external motion measurement section 4 communicatably connected to the motion information obtaining section 2, as shown in Fig. 6.

The external motion measurement section 4 includes an electrocardiograph, a magnetocardiograph, a sphygmoscope and a respirometer, and obtains a measured value of motion of the living tissue using electromagnetism.

This embodiment is the same as the first embodiment except that the following program is executed in step S34.

The motion information obtaining section 2 obtains the motion vector shown in Fig. 4 described above from the ultrasonic image in the different time phase, the stored ultrasonic image and the measured value from the external motion measurement section 4 such as electrocardiograph. Since the electrocardiographic signal of systole/diastole of the heart muscle can be obtained in synchronization with the image measurement, the measured value can be included in the calculation of the motion vector. Specifically, the electrocardiograph can detect an increase or decrease in heart rate. So, when R wave is to be used as a reference timing, even if the predetermined time phase and the different time phase do not occur periodically, the motion vector can be calculated using image data in synchronization with R wave measured by the external motion measurement section 4. The motion extraction calculation section 31 analyzes the directional components of the obtained motion vector (step S34).

This embodiment has the effect of improving the classification accuracy of medical images including those of moving living tissues. Furthermore, this embodiment has the specific effect of enabling calculation of the motion vector even if the predetermined time phase and the different time phase do not occur periodically.

**Fourth Embodiment**

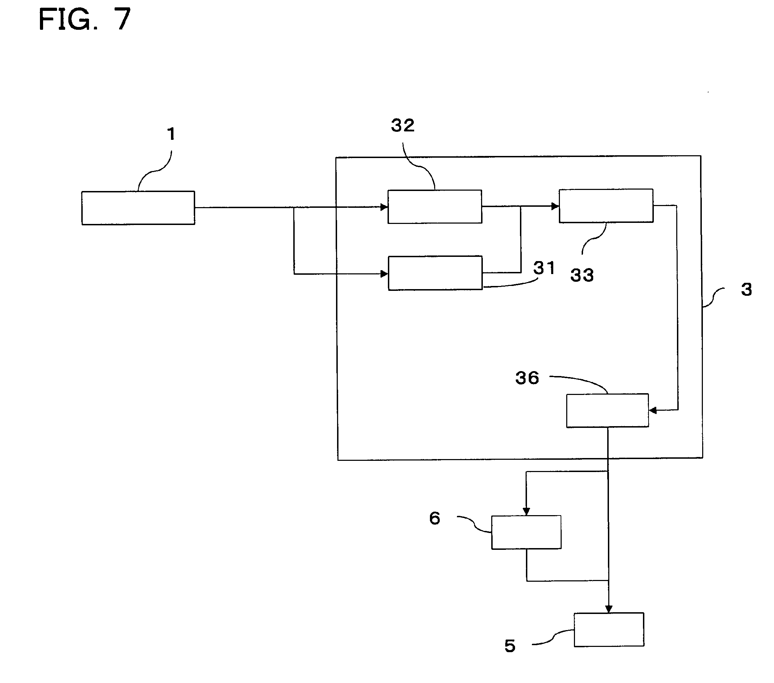

In addition to the configuration of the first embodiment, the medical image processing device further includes a lesion type estimation section 6 communicatably connected to the image recognition calculation section 3, as shown in Fig. 7.

The lesion type estimation section 6 adds diagnostic information given by preliminary image diagnosis to a medical image including a lesion area of the medical images and stores the diagnostic information and the medical image including the lesion area associated with each other.

This embodiment is the same as the first embodiment except that the following program is executed in steps S35 and S36.

The feature extraction calculation section 33 of the image recognition calculation section 3 extracts the feature quantity of each cross section V from the information lj by applying, for example, a method using calculation of basic statistics, such as average or variance, or principal component analysis or independent component analysis. The identification calculation section 36 identifies the basic cross sections in echocardiography from the feature quantity and outputs data of the basic cross section information and the ultrasonic image associated with each other to the lesion type estimation section 6. For example, the lesion type estimation section 6 calculates an index well known as "wall motion csore index." For example, this index is used to determine whether or not the heart muscle in the coronary artery dominance area in coronary artery disease has a myocardial infarction. The lesion type estimation section 6 generates a display format in which disease information obtained using such an index is added to the ultrasonic image (step S35).

The image display 5 displays the index and the ultrasonic image side by side according to the display format. Note that this display step is not necessary when the ultrasonic image is classified and stored in the storage section without being displayed (step S36).

This embodiment has the effect of improving the classification accuracy of medical images including those of moving living tissues. Furthermore, this embodiment has the specific effect of enabling presentation of the disease classification to the tester.

**Fifth Embodiment**

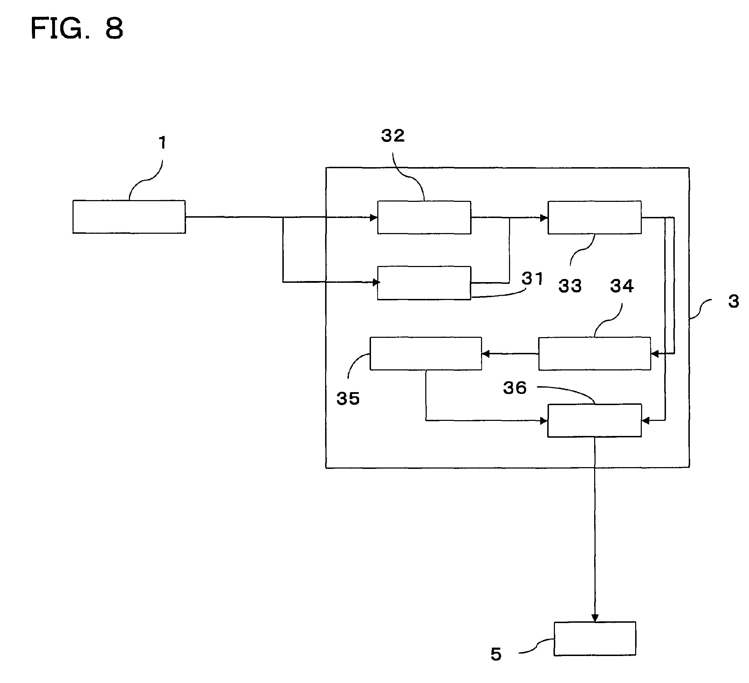

In addition to the configuration of the first embodiment, the medical image processing device further includes, in the image recognition calculation section 3, a learning calculation section 34 communicatably connected to the feature extraction calculation section 33 and a learning data storage section 35 communicatably connected to the learning calculation section 34 and the identification calculation section 36, as shown in Fig. 8.

The learning calculation section 34, having a well known learning algorithm such as neural network, receives the feature quantity output from the feature extraction calculation section 33 and performs learning calculation.

The learning data storage section 35 is a storage device, such as hard disk or memory, for storing learning data calculated by the learning calculation section 34.

The identification calculation section 36 identifies a feature extracted from a newly entered image based on the learning data stored in the learning data storage section 35.

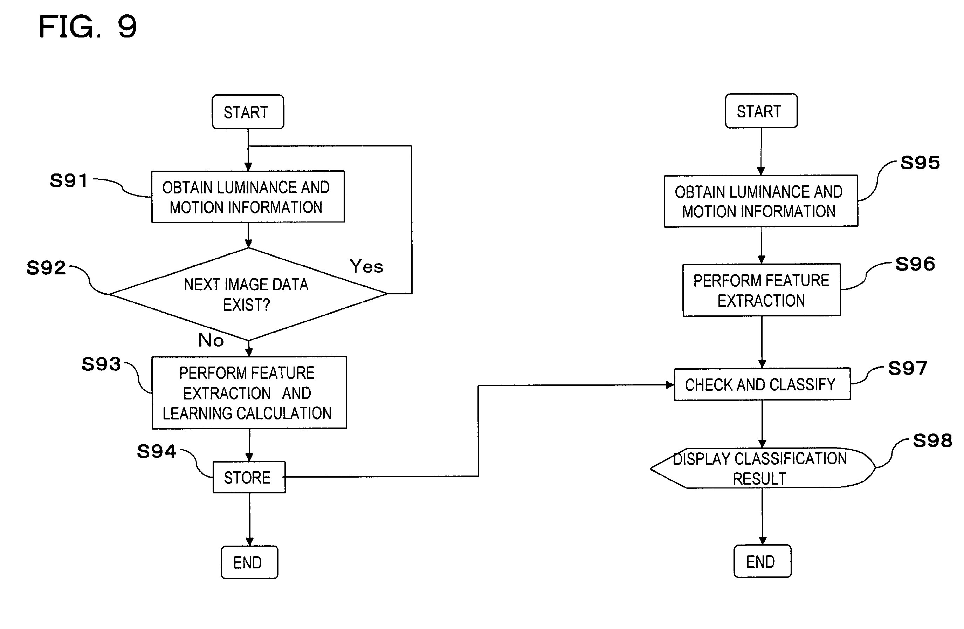

Fig. 9 is a flowchart schematically showing an operation of the medical image processing device in accordance with the fifth embodiment of the invention.

The invention envisages a learning algorithm having a teacher signal, which can be divided into a part of learning with multiple images and a part of recognizing an input image based on the learning data.

In the learning process, first, the luminance extraction calculation section 32 extracts the luminance of a living tissue portion of the image and the motion extraction calculation section 31 extracts the motion vector of the living tissue portion of the image (step S91).

Suppose, for example, luminance is extracted from a sector image. The luminance and motion of the entire image may be calculated, but it requires large amount of calculation. So, for example, calculation may be performed on samples on a grid at regular intervals. For luminance, the luminance extraction calculation section 32 may extract a raw luminance value of the image or may take an average over a certain proximity. Time phase of the image can be freely selected. Commonly, the image with R wave time phase of ECG (electrocardiogram) may be used, the time phase of which is most easily detectable. Also, if the tester sets extraction locations only at portions, such as heart muscle, having a high luminance value, the number of extraction points can be reduced to shorten calculation time. The motion of the heart is calculated by motion extraction calculation at the extraction locations in the motion extraction calculation section 31. For example, a motion calculation method using block-matching method or gradient method is used. The time period over which the motion is calculated is freely settable according to an individual difference among the subjects to be tested. For example, the time period over which the motion is calculated may be a time period during which the amount of motion is largest, such as from end-diastole to end-systole. If a next image data exists, the luminance value and motion are extracted similarly (step S92).

The feature extraction calculation section 33 performs feature extraction calculation on information lj obtained from the image j (step S93).

The feature extraction calculation section 33 extracts the feature of each cross section V by applying, for example, a method using calculation of basic statistics, such as average or variance, or principal component analysis or independent component analysis. The learning calculation section 34 performs learning calculation on the extracted feature. The learned data is stored in the learning data storage section 35, and the learning process ends (step S94).

In the recognition process, first, in a way similar to the learning process, from a medical image input from the medical image generator 1, the luminance extraction calculation section 32 extracts the luminance of a living tissue portion of the image and the motion extraction calculation section 31 extracts the motion vector of the living tissue portion of the image (step S95).

The feature extraction calculation section 33, in a way similar to the learning process, performs feature extraction on the luminance and motion (step S96).

The identification calculation section 36 checks the feature of the learned data against the feature of the input image to classify the medical image into the most similar type of cross section (step S97).

The image display 5 displays the classified type of cross section with the ultrasonic image, and the recognition process ends (step S98).

Furthermore, the type of cross section can be stored associated with the image data.

This embodiment has the effect of improving the classification accuracy of medical images including those of moving living tissues. Furthermore, this embodiment has the specific effect of improving the recognition rate by applying a learning algorithm using luminance and motion as feature quantity, compared with the recognition using only luminance. Furthermore, the quality of the motion can also be classified, which is useful for classifying the heart motion.

**Sixth Embodiment**

A medical image processing device in accordance with a sixth embodiment is implemented in the same hardware configuration as that of the fifth embodiment.

A method in accordance with the sixth embodiment is to perform recognition processing on each local area of the heart muscle rather than the entire image.

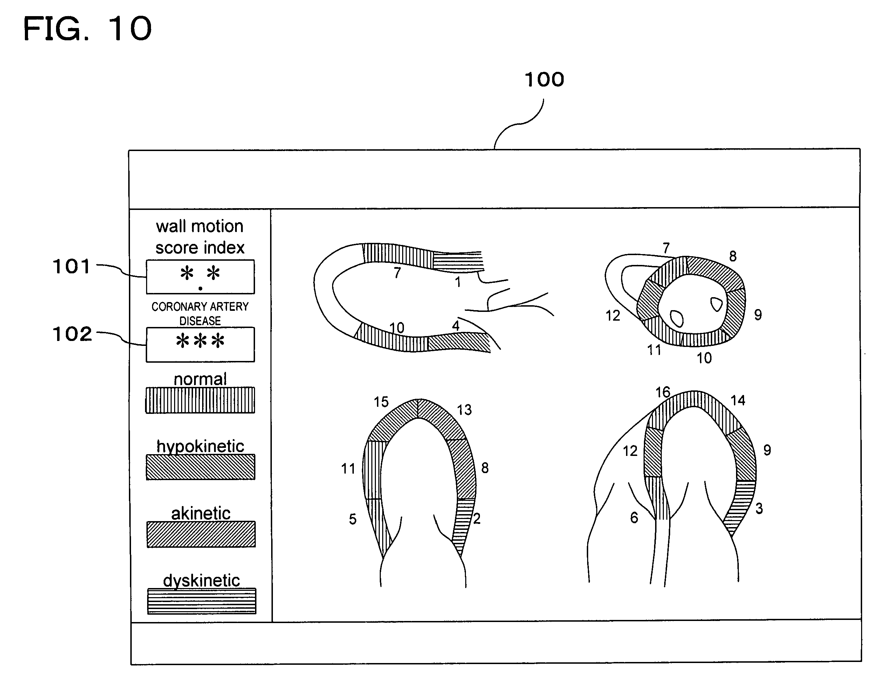

Fig. 10 shows a display example of a stress echo test for explaining the sixth embodiment of the invention.

For example, in the stress echo test, as shown in Fig. 10, the heart muscle is segmented into 16 fractions in a window 90, then the tester makes a visual inspection of the motion of the heart muscle and expresses the inspection result by a score, and then the score is displayed in areas 91, 92 and recorded. The score would be "cannot assess" (no point), "normal" (one point), "hypokinetic" (two points), "akinetic" (three points) or "dyskinetic" (four points). The total sum of these scores are divided by the number of the visualized fractions. Then, the division result is used as "wall motion score index" to totally assess the wall motion. This operation requires the visual inspection for each fraction while switching cross sections, which is very troublesome for the operator. In the sixth embodiment, although probe operation is performed by the tester, the recognition of the type of cross section and the scoring is performed by calculation, which intends to reduce the amount of operation of the operator.

This embodiment is the same as the fifth embodiment except that the following program is executed in steps S91 and S95.

In the learning process, first, the feature extraction calculation section 33 recognizes the type of cross section from the luminance of the image calculated by the luminance extraction calculation section 32 and the motion of the heart calculated by the motion extraction calculation section 31.

Next, the motion information obtaining section 2 segments the muscle heart, divided into partitions, into fractions by existing automatic contour extraction processing or manually.

Next, the luminance extraction calculation section 32 and the motion extraction calculation section 31 place measuring points in the fractions and calculate the luminance and motion in a way similar to the fourth embodiment. Specifically, the luminance extraction calculation section 32 extracts the luminance of a living tissue portion of the image and the motion extraction calculation section 31 extracts the motion vector of the living tissue portion of the image (step S91).

The processing in step 91, that is, the above-described processing is also added to the first step, step 95, of the recognition process.

This embodiment has the effect of improving the classification accuracy of medical images including those of moving living tissues. Furthermore, this embodiment has the specific effect of enabling automatic quantification of abnormality of the motion of the local heart muscle by recognizing the motion for each fraction of the heart muscle, which is difficult for a still image. Furthermore, using information on both the motion and luminance improves the accuracy of the quantification.

**Seventh Embodiment**

In a medical image processing device in accordance with a seventh embodiment, an image search function is added to the image recognition calculation section 3. The remaining part other than the additional function is implemented in the same hardware configuration as that of the fifth embodiment.

The image search function of the seventh embodiment performs image search in testing and performs image search using a thumbnail search window. The method of image search is based on a well known image recognition technique.

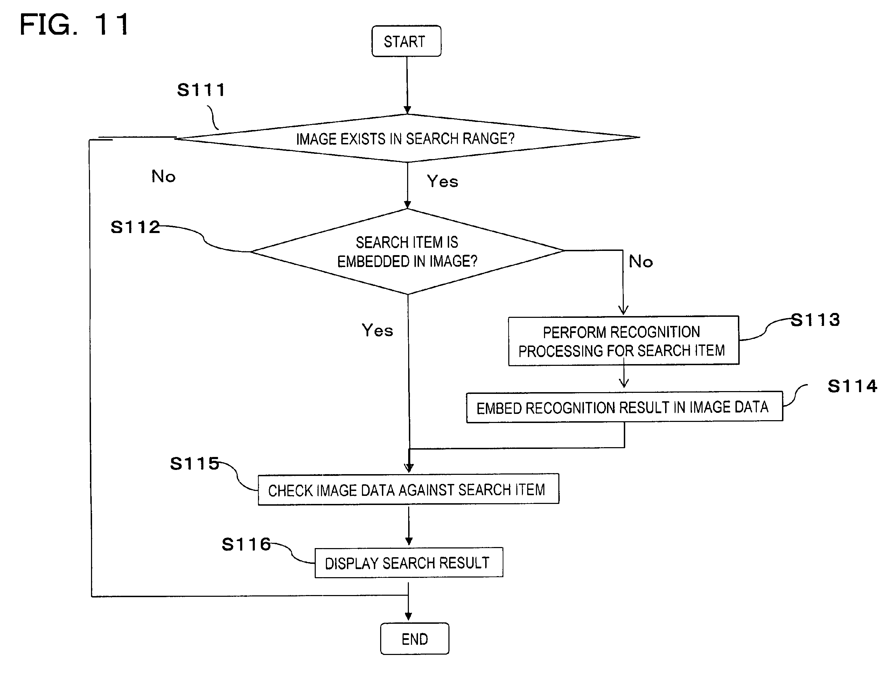

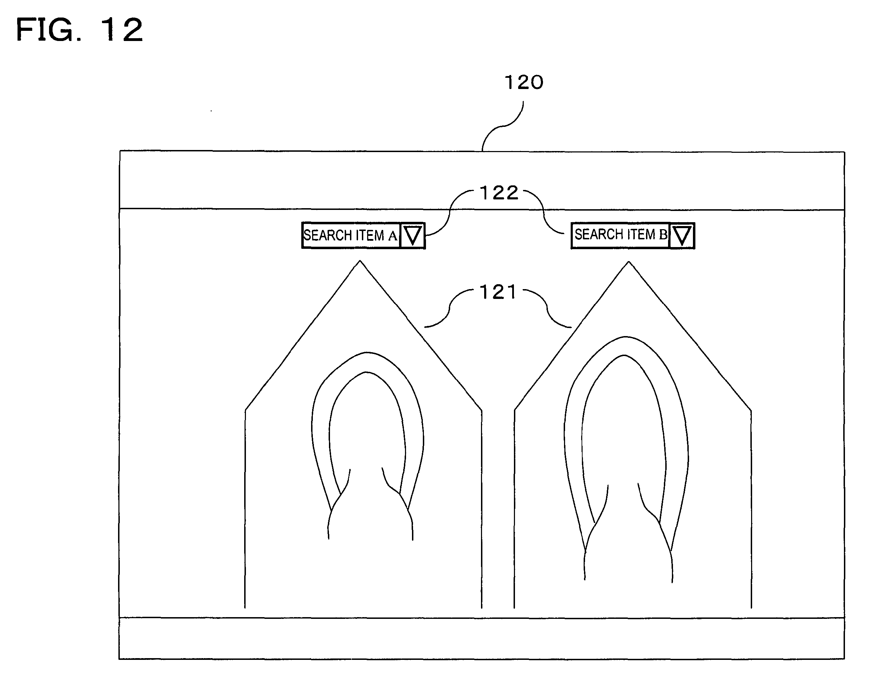

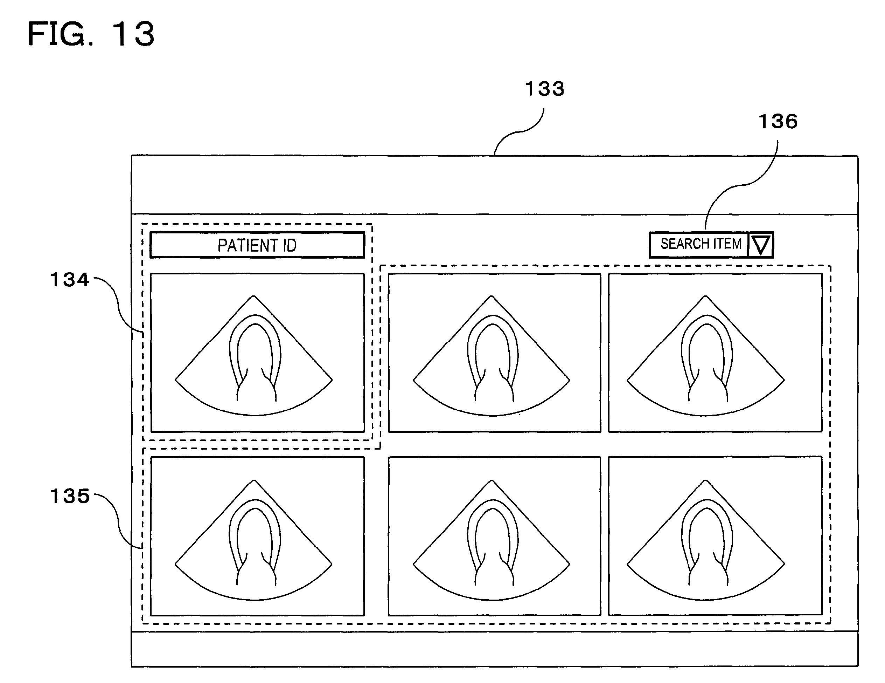

Fig. 11 is a flowchart schematically showing an operation of image search of the medical image processing device in accordance with the seventh embodiment of the invention. Fig. 12 shows a display example in the case that an image most similar to an image from the medical image generator is displayed. Fig. 13 shows a display example in the case that two or more images are similar to an image from the medical image generator.

On starting image search, the image recognition calculation section 3 searches a certain range (for example, of the same patient, the same test date, the same cross section) for an image specified by a search item 122 or 136, and determines whether the targeted image exists or not. The search item 122 or 136 can be freely specified by the tester by operating a software switch or pull-down menu on the screen with a pointing device (step S111).

If an image exists in the search range, the image display 5 displays the image in order for the tester to check whether or not the specified search item is added to the image data (step S112).

If the search item is not added, the image recognition calculation section 3 performs recognition processing for the search item according to the first to fourth embodiments (step S113).

The image recognition calculation section 3 stores the classified type embedded in the image data (step S104). The image recognition calculation section 3 checks the stored image data with the classified type embedded against the specified search item 122 or 136 (step S115).

The image recognition calculation section 3 stores an image data matching the search item 122 or 136 as search result. The image display 5 displays the image data with the specified search item added (step S116).

This embodiment has the effect of improving the classification accuracy of medical images including those of moving living tissues. Furthermore, this embodiment has the specific effect as described below for each display example.

In the display example shown in Fig. 12, a graphical user interface (GUI) 120 in B-mode image testing is shown. For example, in testing a given subject to be tested, specifying apical two chamber view as a left search item A122 and apical four chamber view as a right search item B122 and performing search retrieves and displays stored images for the specified items, allowing the two windows to be compared. Furthermore, specifying a type of motion abnormality as a search item also enables searching for similar lesion. This can reduce the amount of operation of the tester for visually selecting an image from many image data and diagnostic information.

In the display example shown in Fig. 13, a search window 133 using thumbnail images is shown. Suppose, for example, a upper left image 134 is a reference source image of a given subject to be tested. When a search item is specified in a search item 136, a group of candidate images similar to the reference source image 134 with respect to the search item is displayed on candidate window 135. For example, when "wall motion score index" is specified as search item, images having a index close to the index of the reference source image are displayed in the candidate window 135. This facilitates the comparison with an image including a similar lesion or motion, which can improve the quality of test and reduce the amount of operation of the tester.

In the above description, the extraction of the feature quantity is intended for the pixels of overall ultrasonic images. However, the following method performed on sample points on the heart wall may also be used.

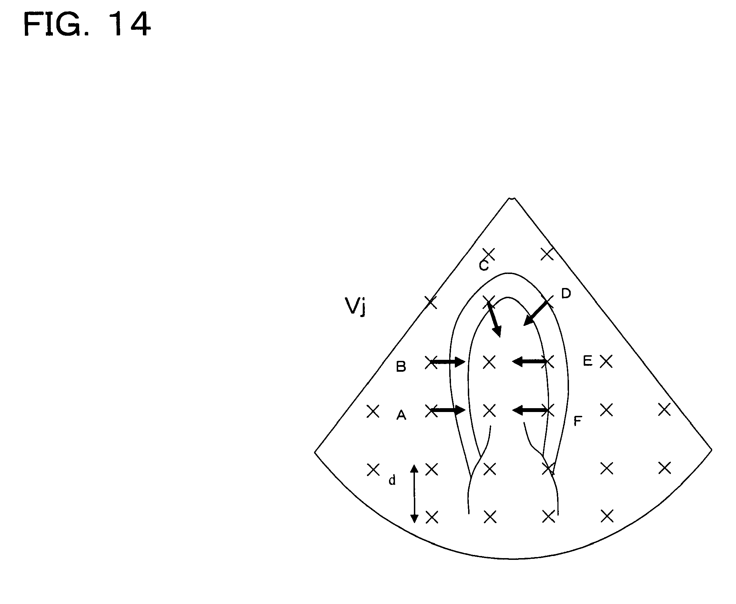

As one specific example, consider the case of calculating the feature quantity of apical two chamber view shown in Fig. 14 from a sector image. Fig. 14 shows an example of vector calculation method different from that of Figs. 2 and 5. First, extraction locations (shown by x) are disposed on a grid at regular intervals all over the sector image (d represents the distance between extraction locations). Then, first motion vectors A to F from the extraction locations overlapping or in proximity to the heart wall or heart chamber in the sector image are defined. For the first motion vectors, respective moving distances and moving directions from diastole to systole of the heart beat are calculated. For example, the relation between the moving distances and moving directions of the first motion vectors A to F is as follows:

| | | |
|---|---|---|
| First vector A | moving distance: $d/2$, | |
| | moving direction: 3 o'clock direction | |
| First vector B | moving distance: $d/2$, | |
| | moving direction: 3 o'clock direction | |
| First vector C | moving distance: $d/2$, | |
| | moving direction: 11 o'clock direction | |
| First vector D | moving distance: $d/2$, | |
| | moving direction: 1 o'clock direction | |
| First vector E | moving distance: $d/2$, | |
| | moving direction: 9 o'clock direction | |
| First vector F | moving distance: $d/2$, | |
| | moving direction: 9 o'clock direction | |

If a measured sector image matches all of the six types, the sector image is classified as apical two chamber view. Even if the sector image matches five of the six types, the sector image is classified as a candidate of apical two chamber view. If the sector image matches four or less of the six types, the sector image may be other than apical two chamber view. So, in this case, vector analysis from the extraction locations is performed for (a) parasternal long axis view, (b) parasternal short-axis view, (d) apical long axis view and (e) apical four chamber view in a way similar to that for (c) apical two chamber view, and which type of image the sector image is classified as is determined.

In the above, the case of performing image processing on the heart has been described. When the target of image processing is a different living tissue from the heart, the time period for extracting the motion should be set as appropriate for the different living tissue.

Also, in the above, the embodiments have been described with reference to the case of applying to a tomography image (B-mode image). In addition, the method can be directly applied to various medical image diagnostic apparatuses for imaging an elasticity imaging image, X-ray image, X-ray CT image and MRI image, or can be applied to a reference image for real-time virtual sonography (RVS) image.

Although the preferred embodiments of the medical image processing device and the like in accordance with the invention have been described with reference to the accompanying drawings, the invention is not limited to these embodiments. It is apparent to the person skilled in the art that various variations and modifications can be conceived without departing from the scope of the technical spirit disclosed herein, and also it is understood that those variations and modifications naturally fall within the technical scope of the invention.

**Description of Reference Numerals and Signs**

1    image information obtaining section,

3    image recognition calculation section

**Claims**

**1.** A medical image processing device, **characterized by** comprising:

an image information obtaining section configured to otain multiple medical images in which a living tissue of a subject to be tested is imaged; and

an image recognition calculation section configured to obtain motion information of the living tissue from pixel values of medical images at different time phases of the obtained multiple medical images and classify the medical images into predetermined types based on the motion information.

**2.** The medical image processing device according to claim 1, comprising a motion information obtaining section configured to obtain motion information of the living tissue from pixel values of local areas of medical images at different time phases of obtained multiple medical images from the image information obtaining section, wherein the image recognition calculation section classifies the medical images into predetermined types based on the motion information obtained from the motion information obtaining section.

**3.** The medical image processing device according to claim 1, further comprising an external motion measurement device, including an electrocardiograph, a magnetocardiograph, a sphygmoscope and a respirometer, configured to obtain a measured value of motion of the living tissue using electromagnetism, wherein the motion information obtaining section obtains motion information of the living tissue from the measured value of motion of the living tissue measured by the external motion measurement device.

**4.** The medical image processing device according to claim 1, further comprising a lesion type estimation section configured to add diagnostic information given by preliminary image diagnosis to a medical image including a lesion area of the medical images and storing the diagnostic information and the medical image including the lesion area associated with each other, wherein the image recognition calculation section classifies the medical images into estimated types of the lesion area based on the medical images including the lesion area and the motion information of the living tissue.

**5.** The medical image processing device according to claim 1, further comprising an extraction time period variable setting section configured to variably set a time period for extracting the motion of the living tissue, wherein the image recognition calculation section classifies the medical images into predetermined types during the variably set time period for extracting the motion of the living tissue.

**6.** The medical image processing device according to claim 1, further comprising an extraction area setting section configured to set an area for extracting the motion of the living tissue to either the entire area or a local area of the targeted image in the ultrasonic diagnostic apparatus, wherein the image recognition calculation section classifies the medical images into predetermined types within the set area for extracting the motion of the living tissue.

**7.** The medical image processing device according to claim 1, wherein the image recognition calculation section classifies the medical images into predetermined types based on morphological information and the motion information of the living tissue.

**8.** The medical image processing device according to claim 1, wherein the image recognition calculation section comprises: a learning calculation section configured to determine a pattern to be used to classify the medical images into predetermined types; a learning data storage section configured to store the determined pattern; and an identification calculation section configured, when an event requiring the update of the pattern exists among subsequent events, to cause the learning calculation section to redetermine the pattern and causing the learning data storage section to update the pattern with the redetermined one and store the updated pattern.

**9.** The medical image processing device according to claim 1, further comprising a image display configured to display the medical images classified into the predetermined types by the image recognition calculation section.

**10.** The medical image processing device according to claim 1,

wherein the image information obtaining section is a ultrasonic diagnostic apparatus comprising: a probe configured to transmit a ultrasonic signal to the subject to be tested and receive a reflected echo signal from the subject to be tested; a probe driver configured to drive the prove to transmit the ultrasonic signal; and a image conversion section configured to convert the reflected echo signal to an ultrasonic image data, and

wherein the image recognition calculation section uses the amplitude of the ultrasonic signal as the morphological information of the living tissue to classify the ultrasonic image into predetermined types based on the motion information and the morphological information of the living tissue.

11. The medical image processing device according to claim 1, wherein the image recognition calculation section displays an image retrieved according to a specified search item.

12. A medical image processing method, **characterized by** comprising:

a step in which an image information obtaining section obtains multiple medical images in which a living tissue of a subject to be tested is imaged; and
a step in which an image recognition calculation section obtains motion information of the living tissue from pixel values of medical images at different time phases of the obtained multiple medical images and classifies the medical images into predetermined types based on the motion information.

FIG. 1

# FIG. 2

(a)  (b)

(c)  (d)  (e)

EP 2 298 176 A1

# FIG. 3

```
                    START
                      │
                      ▼
S31 ─── OBTAIN DATA IN PREDETERMINED TIME PHASE
                      │
                      ▼
S32 ───        STORE DATA
                      │
                      ▼
S33 ─── OBTAIN DATA IN DIFFERENT TIME PHASE
                      │
                      ▼
S34 ───   OBTAIN MOTION INFORMATION
                      │
                      ▼
S35 ───      EXTRACT FEATURE
                      │
                      ▼
S36 ───      DISPLAY IMAGE
                      │
                      ▼
                    END
```

EP 2 298 176 A1

FIG. 4

EP 2 298 176 A1

# FIG. 5

(a)

(b)

(e)

(c)

yjk

xjk

Ajk

EP 2 298 176 A1

# FIG. 6

FIG. 7

EP 2 298 176 A1

FIG. 8

EP 2 298 176 A1

# FIG. 9

START → OBTAIN LUMINANCE AND MOTION INFORMATION (S91) → NEXT IMAGE DATA EXIST? (S92)

NEXT IMAGE DATA EXIST? — Yes → OBTAIN LUMINANCE AND MOTION INFORMATION

NEXT IMAGE DATA EXIST? — No → PERFORM FEATURE EXTRACTION AND LEARNING CALCULATION (S93) → STORE (S94) → END

START → OBTAIN LUMINANCE AND MOTION INFORMATION (S95) → PERFORM FEATURE EXTRACTION (S96) → CHECK AND CLASSIFY (S97) → DISPLAY CLASSIFICATION RESULT (S98) → END

FIG. 10

# FIG. 11

START

S111 — IMAGE EXISTS IN SEARCH RANGE?

No / Yes

S112 — SEARCH ITEM IS EMBEDDED IN IMAGE?

Yes / No

S113 — PERFORM RECOGNITION PROCESSING FOR SEARCH ITEM

S114 — EMBED RECOGNITION RESULT IN IMAGE DATA

S115 — CHECK IMAGE DATA AGAINST SEARCH ITEM

S116 — DISPLAY SEARCH RESULT

END

# FIG. 12

EP 2 298 176 A1

# FIG. 13

133

136

134

135

PATIENT ID

SEARCH ITEM ▽

FIG. 14

<table>
<tr><td colspan="2"><b>INTERNATIONAL SEARCH REPORT</b></td><td>International application No.<br>PCT/JP2009/060043</td></tr>
</table>

| A. CLASSIFICATION OF SUBJECT MATTER |
|---|
| *A61B8/08*(2006.01)i, *A61B6/00*(2006.01)n |

According to International Patent Classification (IPC) or to both national classification and IPC

| B. FIELDS SEARCHED |
|---|

Minimum documentation searched (classification system followed by classification symbols)
A61B8/08, A61B6/00

Documentation searched other than minimum documentation to the extent that such documents are included in the fields searched
Jitsuyo Shinan Koho     1922-1996   Jitsuyo Shinan Toroku Koho   1996-2009
Kokai Jitsuyo Shinan Koho  1971-2009   Toroku Jitsuyo Shinan Koho   1994-2009

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y<br>A | JP 2007-530160 A  (Siemens Medical Solutions USA, Inc.), 01 November, 2007 (01.11.07), Particularly, refer to 28, 29, 33, 36 & US 2005/0251013 A1     & WO 2005/096226 A2 | 1,2,5-11<br>3<br>4 |
| Y | JP 58-180138 A  (Hitachi Medical Corp.), 21 October, 1983 (21.10.83), Particularly, Claim 1 (Family: none) | 3 |
| A | JP 2006-110190 A  (Toshiba Corp.), 27 April, 2006 (27.04.06), Full text; all drawings & US 2006/0083416 A1 | 1-11 |

☒ Further documents are listed in the continuation of Box C.　　☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>23 June, 2009 (23.06.09) | Date of mailing of the international search report<br>07 July, 2009 (07.07.09) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/060043 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2004-313291 A  (Toshiba Corp.), 11 November, 2004 (11.11.04), Full text; all drawings (Family: none) | 1-11 |

Form PCT/ISA/210 (continuation of second sheet) (April 2007)

## INTERNATIONAL SEARCH REPORT

| International application No. |
| --- |
| PCT/JP2009/060043 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 12

    because they relate to subject matter not required to be searched by this Authority, namely:

    The invention as set forth in the above claim is deemed to be concerned with diagnostic methods for human body and thus relates to a subject matter which this International Searching Authority is not required, under the
(Continued to extra sheet)

2. ☐ Claims Nos.:

    because they relate to parts of the international application that do not comply with the prescribed requirements to such an extent that no meaningful international search can be carried out, specifically:

3. ☐ Claims Nos.:

    because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**

the

    ☐ The additional search fees were accompanied by the applicant's protest and, where applicable, payment of a protest fee.

    ☐ The additional search fees were accompanied by the applicant's protest but the applicable protest fee was not paid within the time limit specified in the invitation.

    ☐ No protest accompanied the payment of additional search fees.

Form PCT/ISA/210 (continuation of first sheet (2)) (April 2007)

| INTERNATIONAL SEARCH REPORT | International application No. |
|---|---|
| | PCT/JP2009/060043 |

Continuation of Box No.II-1 of continuation of first sheet(2)

provisions of Article 17(2)(a)(i) of the PCT and Rule 39.1(iv) of the Regulations under the PCT, to search.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002140689 A